(19) Europäisches Patentamt
European Patent Office
Office européen des brevets

(11) EP 4 399 270 B1

(12) **EUROPEAN PATENT SPECIFICATION**

(45) Date of publication and mention of the grant of the patent:
**30.04.2025 Bulletin 2025/18**

(21) Application number: **22769231.6**

(22) Date of filing: **25.08.2022**

(51) International Patent Classification (IPC):
***C11D 3/50*** (2006.01) ***C11D 3/37*** (2006.01)
***D06M 13/217*** (2006.01) ***C11D 17/06*** (2006.01)
***A61K 8/02*** (2006.01) ***A61K 8/86*** (2006.01)
***A61Q 13/00*** (2006.01)

(52) Cooperative Patent Classification (CPC):
**C11D 3/505; A61K 8/0241; A61K 8/86; A61Q 13/00; C11D 3/3707; C11D 17/065;**
A61K 2800/56; D06M 13/005

(86) International application number:
**PCT/EP2022/073725**

(87) International publication number:
**WO 2023/036623 (16.03.2023 Gazette 2023/11)**

(54) **PERFUME PARTICLE**

DUFTSTOFFPARTIKEL

PARTICULE DE PARFUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2021 EP 21196185**

(43) Date of publication of application:
**17.07.2024 Bulletin 2024/29**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **BARR, Helen**
  **deceased (ZZ)**
- **BURGESS, Karl**
  **6708 WH Wageningen (NL)**

(74) Representative: **Reijns, Tiemen Geert Pieter**
**Unilever Patent Group**
**Bronland 14**
**6708 WH Wageningen (NL)**

(56) References cited:
**WO-A1-2019/025216** **WO-A1-2020/209908**
**CA-A1- 2 762 589** **US-A1- 2009 169 500**
**US-B2- 6 624 136**

- **WEI MIN ET AL: "Polymer carriers for controlled fragrance release", vol. 7, no. 8, 1 August 2020 (2020-08-01), pages 082001, XP055895458, Retrieved from the Internet <URL:https://iopscience.iop.org/article/10.1088/2053-1591/aba90d/pdf> DOI: 10.1088/2053-1591/aba90d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 399 270 B1

## Description

### Field of the Invention

The present invention relates to perfume particles comprising ingredients comprising ethoxylate units derived from biomass.

### Background of the Invention

Fragrance is an important aspect of the laundry process. Consumers often associate fragrance with cleanliness or simply enjoy the smell; accordingly, many laundry products comprise perfumes. However, the desired quantity of perfume varies from consumer to consumer. Consequently, perfume particles have been developed to allow consumers to tailor their perfume experience based on their personal preferences.

Perfume particles generally include ingredients such as fragrance materials and a carrier material. Common carrier materials include ingredients comprising ethoxylate groups, such as alcohol ethoxylates and polyethylene glycol ingredients, such as disclosed in WO 2019/025216 A1.

The colour stability or aesthetics of fragrance particles are an important characteristic of fragrance particles. If the particles change their aesthetic characteristics over time, the consumers may view the products as inferior.

It has been observed that fragrance particles comprising ingredients comprising ethoxylate groups can demonstrate colour changes under certain storage conditions. Therefore, there is a need to ensure colour stability overtime for fragrance particles.

### Summary of the Invention

We have found that perfume particles comprising at least 10 wt.% of a carrier material which comprises at least one ethoxylate unit derived from biomass provides enhanced colour stability.

The present invention relates to a perfume particle composition according to claim 1.

The invention further relates to a method of preparing said perfume particle composition, according to claim 11.

The invention additionally relates to a use of said perfume particle as described herein to reduce the carbon footprint of the perfume particle, according to claim 12.

### Detailed Description

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The term 'virgin fossil fuels' refers to fossil fuel sources (coal, crude oil, natural gas) which have not previously been used for any other purpose, i.e. have not been burnt for energy, or are not the waste gas from an industrial process.

The term 'biomass' refers to organic matter, such as plants and does not include virgin fossil fuels.

The perfume particles described herein comprise carrier materials comprising at least one ethoxylate unit derived from biomass. To obtain these carrier materials from biomass the biomass must be converted to ethanol and then used to produce a carrier material for use in perfume particles. The conversion of biomass to ethanol and then ethanol to a carrier material may occur in one continuous process or may occur in several stages and/or at several different locations.

The biomass may be any suitable organic material. Preferably the biomass is plant biomass, more preferably cellulosic materials.

There are various methods of converting biomass to ethanol. One particularly method involves hydrolysing the cellulose to simple sugars such as sucrose, fructose or glucose, then fermenting the simple sugars to produce ethanol. The hydrolysis is preferably performed using concentrated acids, dilute acids or enzymes. The fermentation is preferably performed by yeast in the absence of oxygen.

Once the ethanol is produced, it is preferably purified by fractional distillation. The ethanol is then used to produce

a carrier material.

By carrier is meant a solid material which provides the solid structure of the perfume particle. The compositions described herein preferably comprise at least 50 wt.% carrier materials, preferably 65 wt.%, more preferably 80 wt.% and most preferably at least 90 wt.% carrier materials, by weight of the composition. Preferably less than 98 wt. % carrier materials. This refers to the carrier material which comprises at least one ethoxylate unit derived from biomass and any additional carrier materials.

Generally, carrier materials may be any material which disperses, dissolves, disintegrates or solubilises in water. The composition my comprise one carrier material or a combination of different carrier materials.

The perfume particles comprise at least 10 wt.% of a carrier material which comprises at least one ethoxylate unit derived from biomass, more preferably at least 20 wt. %, even more preferably at least 50 wt.%, most preferably at least 60 wt.% and preferably less than 98 wt. %.

The carrier material which comprises at least one ethoxylate unit derived from biomass on average comprises at least 50 wt.% of the ethoxylate units derived from biomass, more preferably at least 70 wt.%, most preferably all the ethoxylate units in the molecule are derived from biomass. The carrier material which comprises at least one ethoxylate unit derived from biomass may also comprise ethoxylate groups derived from virgin fossil fuels, however this is not preferable. Preferably, less than 50 wt. %, more preferably less than 10 wt.% of the ethoxylate groups are derived directly from virgin fossil fuels.

To produce ethoxylates from biomass, first ethanol produced as outlined above is dehydrated to ethylene. The dehydration of ethanol to ethylene is a common industrial process. The ethylene is then oxidised to form ethylene oxide.

Depending on the desired carrier material, different routes are available.

If an alcohol ethoxylate is desired, the ethylene oxide can be reacted with a long chain fatty alcohol via a polymerisation type reaction. This process is commonly referred to as ethoxylation and gives rise to alcohol ethoxylates. Preferably the long chain fatty alcohol comprises carbon from non-virgin fossil fuel sources, more preferably a bio-mass source. More preferably the long chain fatty alcohol comprises only carbon from a plant source.

If a polyethylene glycol is desired, the ethylene oxide can be polymerised, for example in the presence of water and a catalyst to yield a polyethylene glycol chain.

Preferably all carbons within the carrier material ingredient molecule are derived from a bio-mass source, more preferably a plant source.

Preferred ethoxylated materials include: fatty acid ethoxylates, fatty amine ethoxylates, fatty alcohol ethoxylates, nonylphenol ethoxylates, alkyl phenol ethoxylate, amide ethoxylates, Sorbitan(ol) ester ethoxylates, glyceride ethoxylates (castor oil or hydrogenated castor oil ethoxylates) and mixtures thereof.

Preferably the carrier materials comprising at least one ethoxylate unit derived from biomass are selected from alcohol ethoxylates, polyethylene glycols and combinations thereof.

Alcohol ethoxylates:

Alcohol ethoxylates preferably have the general formula:

$$R^1O(R^2O)_xH$$

$R^1$ = hydrophobic moiety.
$R^2$ = $C_2H_4$ or mixture of $C_2H_4$ and $C_3H_6$ units
x = 4 to 120

$R^1$ preferably comprises 8 to 25 carbon atoms and mixtures thereof, more preferably 10 to 20 carbon atoms and mixtures thereof most preferably 12 to 18 carbon atoms and mixtures thereof. Preferably $R^1$ is selected from the group consisting of primary, secondary and branched chain saturated and/or unsaturated hydrocarbon groups comprising an alcohol, carboxy or phenolic group. Preferably $R^1$ is a natural alcohol.

$R^2$ preferably comprises at least 50% $C_2H_4$, more preferably 75% $C_2H_4$, most preferably $R^2$ is $C_2H_4$. Preferably $R^2$ comprises carbons from biomass.

x is preferably 8 to 90 and most preferably 30 to 90.

Polyethylene glycols:

Polyethylene glycols (PEGs) have a general formula:

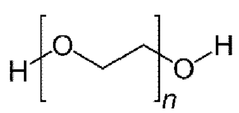

The weight average molecular weight of the PEG is preferably 2000 to 20000, more preferably 3000 to 15000, most preferably 3000 to 11000.

The PEG may solely comprise ethoxylates derived from biomass or may comprise a mixture of ethoxylates derived from biomass and ethoxylates derived from other carbon sources.

The use of a carrier material comprising at least one ethoxylate derived from biomass enhances the colour stability of the perfume particles.

The perfume particles may comprise an additional carrier or a combination of additional carriers. The additional carrier materials may be selected from the group consisting of: non-biomass derived synthetic polymers (e g, polyethylene glycol, ethylene oxide/propylene oxide block copolymers, polyvinyl alcohol, polyvinyl acetate, and derivatives thereof), proteins (e.g., gelatin, albumin, casein), saccharides (e.g. dextrose, fructose, galactose, glucose, isoglucose, sucrose), polysaccharides (e.g., starch, xanthan gum, cellulose, or derivatives thereof), water-soluble or water dispersible fillers (e.g. sodium chloride, sodium sulfate, sodium carbonate/bicarbonate, zeolite, silica, clay), vegetable soap (e.g. coconut soap beads or palm soap), non-biomass derived ethoxylated non-ionic surfactants (having a formula $R_1O(R_2O)xH$, wherein $R_1$ preferably comprises 12 to 20 carbon atoms, $R_2$ is $C_2H_4$ or mixture of $C_2H_4$ and $C_3H_6$ units and x = 8 to 120), urea and combinations thereof. By non-biomass it is meant that no carbon atoms are derived from biomass.

Examples of suitable carrier materials include: water soluble organic alkali metal salt, water soluble inorganic alkaline earth metal salt, water soluble organic alkaline earth metal salt, water soluble carbohydrate, water soluble silicate, water soluble urea, starch, xanthan gum, dextrose, clay, water insoluble silicate, citric acid carboxymethyl cellulose, fatty acid, fatty alcohol, glyceryl diester of hydrogenated tallow, glycerol, non-biomass polyvinyl alcohol, non-biomass non-ionic surfactants sold under the trade name Lutensol ex. BASF and combinations thereof.

Preferred additional carrier materials may be selected from the group consisting of non-biomass derived synthetic polymers (e g, polyethylene glycol, ethylene oxide/propylene oxide block copolymers, polyvinyl alcohol, polyvinyl acetate, and derivatives thereof), polysaccharides (e.g., starch, xanthan gum, cellulose, or derivatives thereof), saccharides (e.g, dextrose, fructose, galactose, glucose, isoglucose, sucrose), vegetable soap (e.g. coconut soap beads or palm soap), non-biomass derived ethoxylated non-ionic surfactants (having a formula $R_{10}(R_2O)xH$, wherein $R^1$ preferably comprises 12 to 20 carbon atoms, $R^2$ is $C_2H_4$ or mixture of $C_2H_4$ and $C_3H_6$ units and x = 8 to 120) and combinations thereof.

More preferably additional carriers are selected from starch, dextrose, coconut soap beads, palm soap and combinations thereof.

Saccharides are molecular compounds comprising carbon, hydrogen and oxygen. For the purposes of this invention a saccharide is defined as comprising one to ten monosaccharide units and mixtures thereof. In other words either a monosaccharide or an oligosaccharide or mixtures thereof. An oligosaccharide is a short saccharide polymer, typically considered in the art to comprise between two and ten monosaccharides units. It is preferred that a saccharide comprises 1 to 5 monosaccharide units, more preferably 1 to 4 monosaccharide units, most preferably the saccharide comprises monosaccharides, disaccharides or mixtures thereof. Disaccharides are the product of a reaction between two monosaccharides. They may be formed from two identical monosaccharides or two different monosaccharides. Examples of disaccharides include: sucrose, maltose, lactose. Monosaccharides are simple sugar units having the general formula $(CH_2O)_n$. Commonly n is 3, 5 or 6. According, monosaccharides can be classified by the number n, for example: trioses (e.g. glyceraldehyde), pentoses (e.g. ribose) and hexoses (e.g. fructose, glucose and galactose). Some monosaccharides may be substituted with additional functional groups, e.g. Glucosamine, others may have undergone deoxgenation and lost an oxygen atom e.g. deoxyribose. Therefore, the general chemical formulae can vary slightly depending on the monosaccharide.

Preferred monosaccharides for the present invention are hexose molecules (n=6). Hexose molecules all have the same molecular formula, however, have a different structural formula, i.e. are structural isomers. It is preferred that the hexose comprises a 6-membered ring, opposed to a 5 membered ring. Glucose and galactose have 6-membered rings. In a preferred embodiment the hexose monosaccharide is glucose. Glucose is a chiral molecule, having a mixture of D and L stereo isomers. Particularly preferably, the glucose of the present invention is the D isomer of glucose, also known as dextrose.

Preferably a saccharide material used in the present invention is anhydrous, i.e. free of any water. For example, dextrose monohydrate contains one molecule of water whereas anhydrous dextrose contains none.

Non-limiting examples of suitable saccharides for the present invention are: C*Dex ex Cargill, Treha ex Cargill, Anhydrous Dextrose ex Foodchem.

When a saccharide is used in the present invention, it may be preferable to include bitter material such as Bitrex ex Johnson Matthey Fine Chemicals, due to the sweetness of the saccharide.

The percentage modern carbon (pMC) level is based on measuring the level of radiocarbon (C14) which is generated in the upper atmosphere from where it diffuses, providing a general background level in the air. The level of C14, once captured (e.g. by biomass) decreases over time, in such a way that the amount of C14 is essentially depleted after 45,000 years. Hence the C14 level of fossil-based carbons, as used in the conventional petrochemical industry is virtually

zero.

A pMC value of 100% would indicate that 100% of the carbon came from plants or animal by-products (biomass) living in the natural environment and a value of 0% would mean that all the carbon was derived from petrochemicals, coal and other fossil sources. A value between 0 and 100% would indicate a mixture. The higher the value, the greater the proportion of naturally sourced components in the material.

In one embodiment, the carrier material comprising at least one ethoxylate derived from biomass preferably has a pMC of 50% to 100%, preferably 75% to 100%, most preferably 90% to 100%.

The pMC provides a measurable indication of the carbon origins. The use of fossil fuel based carbon alters the pMC in a measurable way, pMC can therefore be used as an eco-marker in products.

The pMC level can be determined using the % Biobased Carbon Content ASTM D6866-20 Method B, using a National Institute of Standards and Technology (NIST) modern reference standard (SRM 4990C). Such measurements are known in the art and are performed commercially, such as by Beta Analytic Inc. (USA). The technique to measure the C14 carbon level is known since decades and most known from carbon-dating archaeological organic findings.

The compositions of the present invention comprise perfume ingredients i.e. free oil perfume or non-confined perfumes and/or perfume microcapsules.

The compositions of the present invention further comprise one or more perfume compositions. The perfume compositions may be in the form of a mixture of free perfume compositions or a mixture of encapsulated and free oil perfume compositions.

Preferably the compositions of the present invention comprise 0.5 to 20 wt.% perfume ingredients, more preferably 1 to 15 wt.% perfume ingredients, most preferably 2 to 10 wt. % perfume ingredients. By perfume ingredients it is meant the combined free perfume and any encapsulated perfume.

Useful perfume components may include materials of both natural and synthetic origin. They include single compounds and mixtures. Specific examples of such components may be found in the current literature, e.g., in Fenaroli's Handbook of Flavor Ingredients, 1975, CRC Press; Synthetic Food Adjuncts, 1947 by M. B. Jacobs, edited by Van Nostrand; or Perfume and Flavor Chemicals by S. Arctander 1969, Montclair, N.J. (USA). These substances are well known to the person skilled in the art of perfuming, flavouring, and/or aromatizing consumer products.

Particularly preferred perfume components are blooming perfume components and substantive perfume components. Blooming perfume components are defined by a boiling point less than 250°C and a LogP greater than 2.5. Substantive perfume components are defined by a boiling point greater than 250°C and a LogP greater than 2.5. Preferably a perfume composition will comprise a mixture of blooming and substantive perfume components. The perfume composition may comprise other perfume components.

It is commonplace for a plurality of perfume components to be present in a free oil perfume composition. In the compositions for use in the present invention it is envisaged that there will be three or more, preferably four or more, more preferably five or more, most preferably six or more different perfume components. An upper limit of 300 perfume ingredients may be applied.

Free perfume may preferably be present in an amount from 0.01 to 20 wt. %, more preferably 0.1 to 15 wt.%, more preferably from 0.1 to 10 wt.%, even more preferably from 0.1 to 6.0 wt.%, most preferably from 0.5 to 6.0 wt. %, based on the total weight of the composition.

Preferably some of the perfume components are contained in a microcapsule. Suitable encapsulating materials may comprise, but are not limited to; aminoplasts, proteins, polyurethanes, polyacrylates, polymethacrylates, polysaccharides, polyamides, polyolefins, gums, silicones, lipids, modified cellulose, polyphosphate, polystyrene, polyesters or combinations thereof.

Perfume components contained in a microcapsule may comprise odiferous materials and/or pro-fragrance materials.

Particularly preferred perfume components are as described for free perfumes.

Encapsulated perfume may preferably be present in an amount from 0.01 to 20 wt.%, more preferably 0.1 to wt.15 %, more preferably from 0.1 to 10 wt.%, even more preferably from 0.1 to 9.0 wt.%, most preferably from 0.5 to 8.0 wt.%, based on the total weight of the composition.

Colour may optionally be provided to the perfume particles by the addition of one or more colorants. The colorant comprises one or more dyes and/or pigments. The pigment/dye may be any colour. These may be substantive or nonsubstantive dyes/pigments. For substantive dyes/pigments a blue or violet colour is preferred. A preferred level is one where the colour is discernible to the consumer and aesthetically pleasing. The laundry products may be a plurality of colours.

Pigments may be selected from inorganic and organic pigments, most preferably the pigments are organic pigments.

Pigments are described in Industrial Inorganic Pigments edited by G. Buxbaum and G. Pfaff (3rd edition Wiley-VCH 2005). Suitable organic pigments are described in Industrial Organic Pigments edited by W. Herbst and K.Hunger (3rd edition Wiley-VCH 2004). Pigments are listed in the colour index international © Society of Dyers and Colourists and

American Association of Textile Chemists and Colorists 2002.

Pigments are practically insoluble coloured particles, preferably they have a primary particle size of 0.02 to 10μm, where the distance represent the longest dimension of the primary particle. The primary particle size is measured by scanning electron microscopy. Most preferably the organic pigments have a primary particle size between 0.02 and 0.2 μm.

By practically insoluble we mean having a water solubility of less than 500 part per trillion (ppt), preferably 10 ppt at 20°C with a 10 wt% surfactant solution.

Organic pigments are preferably selected from monoazo pigments, beta-naphthol pigments, naphthol AS pigments, benzimidazolone pigments, metal complex pigments, isoindolinone and isoindoline pigments, phthalocyanine pigments, quinacridone pigments, perylene and perinone pigments, diketopyrrolo-pyrrole pigments, thioindigo pigments, anthraquinone pigments, anthrapyrmidine pigments, flavanthrone pigments, anthanthrone pigments, dioxazine pigments and quinophthalone pigments.

Preferred pigments are pigment green 8, pigment blue 28, pigment yellow 1, pigment yellow 3, pigment orange 1, pigment red 4, pigment red 3, pigment red 22, pigment red 112, pigment red 7, pigment brown 1, pigment red 5, pigment red 68, pigment red 51, pigment 53, pigment red 53:1, pigment red 49, pigment red 49:1, pigment red 49:2, pigment red 49:3, pigment red 64:1, pigment red 57, pigment red 57:1, pigment red 48, pigment red 63:1, pigment yellow 16, pigment yellow 12, pigment yellow 13, pigment yellow 83, pigment orange 13, pigment violet 23, pigment red 83, pigment blue 60, pigment blue 64, pigment orange 43, pigment blue 66, pigment blue 63, pigment violet 36, pigment violet 19, pigment red 122, pigment blue 16, pigment blue 15, pigment blue 15:1, pigment blue 15:2, pigment blue 15:3, pigment blue 15:4, pigment blue 15:6, pigment green 7, pigment green 36, pigment blue 29, pigment green 24, pigment red 101:1, pigment green 17, pigment green 18, pigment green 14, pigment brown 6, pigment blue 27 and pigment violet 16.

Cosmenyl Green, Cosmenyl Yellow, Cosmenyl Blue and Cosmenyl Red are preferred commercially available pigments.

Dyes are described in Industrial Dyes edited by K.Hunger 2003 Wiley-VCH ISBN 3-527-30426-6.

Dyes for use in the current invention are selected from cationic, anionic and non-ionic dyes.

The dyes may be alkoxylated. Alkoxylated dyes are preferably of the following generic form: Dye-$NR_1R_2$. The $NR_1R_2$ group is attached to an aromatic ring of the dye. $R^1$ and $R^2$ are independently selected from polyoxyalkylene chains having 2 or more repeating units and preferably having 2 to 20 repeating units. Examples of polyoxyalkylene chains include ethylene oxide, propylene oxide, glycidol oxide, butylene oxide and mixtures thereof.

Preferably the dye is selected from acid dyes; disperse dyes and alkoxylated dyes.

Most preferably the dye is an anionic or non-ionic dye. It is even more preferred that the dye is a non-ionic dye.

Preferably the dye is selected from those having: anthraquinone; mono-azo; bis-azo; xanthene; phthalocyanine; and, phenazine chromophores. More preferably the dye is selected from those having: anthraquinone and, mono-azo chromophores.

The dye may be any colour, preferably the dye is blue, violet, green or red.

Preferably the dye is selected from: acid blue 80, acid blue 62, acid violet 43, acid green 25, direct blue 86, acid blue 59, acid blue 98, direct violet 9, direct violet 99, direct violet 35, direct violet 51, acid violet 50, acid yellow 3, acid red 94, acid red 51, acid red 95, acid red 92, acid red 98, acid red 87, acid yellow 73, acid red 50, acid violet 9, acid red 52, food black 1, food black 2, acid red 163, acid black 1, acid orange 24, acid yellow 23, acid yellow 40, acid yellow 11, acid red 180, acid red 155, acid red 1, acid red 33, acid red 41, acid red 19, acid orange 10, acid red 27, acid red 26, acid orange 20, acid orange 6, sulphonated Al and Zn phthalocyanines, solvent violet 13, disperse violet 26, disperse violet 28, solvent green 3, solvent blue 63, disperse blue 56, disperse violet 27, solvent yellow 33, disperse blue 79:1.

The dye may be covalently bound to polymeric species.

The perfume particles may additionally comprise fabric care ingredients. Such fabric care ingredients include silicones, low levels of quaternary ammonium compounds, ester oils and the like.

The compositions of the present invention may contain further optional laundry ingredients. Such ingredients include colourants, preservatives, pH buffering agents, perfume carriers, hydrotropes, polyelectrolytes, anti-shrinking agents, anti-oxidants, anticorrosion agents, drape imparting agents, anti-static agents, ironing aids, antifoams, colorants, pearlisers and/or opacifiers, natural oils/extracts, processing aids, e.g. electrolytes, hygiene agents, e.g. anti-bacterials and antifungals, thickeners and skin benefit agents.

In one embodiment of the present invention is provided a method of preparing said perfume particle composition, wherein the method comprises the steps of:

i. Obtaining a carrier ingredient comprising at least one ethoxylate unit derived from biomass;
ii. Melting said carrier material;
iii. Adding the perfume ingredients;
iv. Shaping and cooling the melt.

The perfume particles may be in any solid form, for example: powder, pellet, tablet, prill, pastille or extrudate. Preferably the composition in the form of a pastille or extrudate. Pastilles can, for example, be produced using ROTOFORMER Granulation Systems ex. Sandvick Materials.

The perfume particle compositions of the present invention may be formed from a melt. The solid composition can for example, be formed into particles by: Pastillation e.g. using a ROTOFORMER ex Sandvick Materials, extrusion, prilling, by using moulds, casting the melt and cutting to size or spraying the melt.

An example manufacturing process may involve melting the carrier material (including the carrier material comprising at least one ethoxylate unit derived from biomass) at a temperature above the melting point of that carrier material, preferably at least 2°C above the melting point of the carrier material, more preferably at least 5°C above the melting point of the carrier material, preferably not more than 20°C above the melting point of the carrier material. Where more than one carrier materials are used, the melting point is considered to be the highest of the melting points of the individual materials. Once melted, perfume and other ingredients may be mixed into the compositions. This is followed by a process in which the melt in cooled and shaped, e.g. extrusion or pastillation.

The perfume particle compositions of the present invention are preferably homogeneously structured. By homogeneous, it is meant that there is a continuous phase throughout the solid product. There is not a core and shell type structure. Any particles present such as perfume microcapsules will be distributed within the continuous phase. The continuous phase is provided predominately by the carrier materials.

The perfume particle compositions may be any shape or size suitable for dissolution in the laundry process. Preferably, each individual particle of the solid composition has a mass of between 0.95mg to 5 grams, more preferably 0.01 to 1 gram and most preferably 0.02 to 0.5 grams. Preferably each individual particle has a maximum linear dimension in any direction of 10 mm, more preferably 1-8 mm and most preferably a maximum linear dimension of 4-6 mm. The shape of the particles may be selected for example from spherical, hemispherical, compressed hemispherical, lentil shaped, oblong, or planar shapes such as petals. A preferred shape for the particles is hemispherical, i.e. a dome shaped wherein the height of the dome is less than the radius of the base. When the particles are compressed hemispherical, it is preferred that diameter of the substantially flat base provides the maximum linear dimension and the height of the particle is 1-5mm, more preferably 2-3mm. The dimensions of the particles of the present invention can be measured using Calipers.

Preferably the perfume particles are packaged in a container. When the container is plastic, preferably the container comprises recycled plastic, in particular PCR. "post-consumer resin (PCR)" typically means plastic that has been collected via established consumer recycling streams, sorted, washed and reprocessed, for example into pellets.

In one aspect of the present invention is provided the use of an ingredient comprising at least one ethoxylate derived from biomass to improve the colour stability of the perfume particles. Improved colour stability can be measured by colour change. The more stable, the smaller the colour change. The measurement can be made simply by visual assessment or can be measured using UV Vis spectrometry such as the spectrometers produced by X-rite, in particular the model VS450. A colourant is not required to see a colour change. A colour change may simply be the yellowing of a white (uncoloured) perfume particle.

Additionally, the present invention provides a use of perfume particles as described herein to reduce the carbon footprint of a product, by using biomass rather than virgin fossil fuels. Biomass is a renewable source and contributes to a circular carbon economy.

The use of an ingredient comprising at least one ethoxylate derived from biomass provides the consumer with a tangible eco marker in the product. Accordingly in one aspect of the present invention is provided a use of an ingredient comprising at least one ethoxylate unit derived from biomass as a tangible eco marker in a perfume particle composition.

## Examples

The effect of using biomass derived carrier materials, rather than virgin petrochemical derived materials is demonstrated herein.

*Table 1: Formulations:*

| | Comparative A | Example 1 |
|---|---|---|
| PEG 8000 (petrochemical)[1] | 65 | - |
| PEG 8000 (biomass)[2] | - | 65 |
| Anhydrous Dextrose[3] | 27 | 27 |
| Free perfume | 6 | 6 |

(continued)

| | Comparative A | Example 1 |
|---|---|---|
| Perfume microcapsules[4] | 2 | 2 |
| PEG 8000 (petrochemical)[1] - All ethoxylate groups are derived from petrochemical sources<br>PEG 8000 (biomass)[2] - All ethoxylate groups are derived from plant biomass<br>Anhydrous Dextrose[3] - C*Dex ex Cargill<br>Perfume microcapsules[4] - weight as supplied | | |

The PEG was heated in a mixing vessel, with stirring, until molten and homogeneous. The dextrose was then slowly added with stirring. Stirring was maintained during the addition of the fragrance, followed by the encapsulated fragrance. The mix was then dropped onto a cold stainless steel surface using a 2 ml pipet and allowed to cool.

To test the colour stability the perfume particles were stored at 50°C. Colour measurements were taken at 0 weeks to provide a baseline, 4 weeks, 8 weeks and 13 weeks. Colour was assessed by the ΔE value, comparing the aged sample to the 0 weeks sample. ΔE was calculated using the CIELAB colour space, wherein each colour has an L*, a* and b* value and

$$\Delta E^*_{ab} = \sqrt{}(L^*_2-L^*_1)+ (a^*_2-a^*_1)+ (b^*_2-b^*_1)$$

The colour assessment was performed using an X-rite VS450 colour spectrometer.

Table 2: Colour stability results

| | **Δ E value** | |
|---|---|---|
| **Weeks stored at 50°C** | **Comparative A** | **Example 1** |
| 0 | 0 | 0 |
| 4 | 8.83 | 5.81 |
| 8 | 12.31 | 7.44 |
| 13 | 14.77 | 9.11 |

As demonstrated, the perfume particles comprising PEG derived from biomass have a significantly smaller ΔE, demonstrating a smaller colour change and therefore a more stable perfume particle.

## Claims

**1.** A perfume particle composition comprising:

a) at least 10 wt.% of a carrier material selected from alcohol ethoxylates, polyethylene glycols and combinations thereof, which comprises at least one ethoxylate unit derived from biomass; and
b) perfume ingredients

wherein at least 50 wt. % of the ethoxylate units in ingredient a) are derived from plant biomass.

**2.** A perfume particle according to claim 1, wherein less than 10 wt.%, of the ethoxylate units in ingredient a) are obtained directly from virgin fossil fuel sources.

**3.** A perfume particle according to any preceding claim, wherein ingredient a) is an alcohol ethoxylate.

**4.** A perfume particle according to any preceding claim, wherein ingredient a) is a polyethylene glycol having a molecular weight of 2000 to 20000.

**5.** A perfume particle according to any preceding claim, wherein the biomass is cellulosic materials.

6. A perfume particle according to any preceding claim, wherein the carrier material comprising at least one ethoxylate derived from biomass has a percentage modern carbon (pMC) of 50% to 100%, which is determined according to the specification in the description.

7. A perfume particle according to any preceding claim, wherein the perfume particle comprises an additional carrier material, selected from: non-biomass derived synthetic polymers, proteins, saccharides, polysaccharides, water-soluble or water dispersible fillers, vegetable soap, non-biomass derived ethoxylated non-ionic surfactants, urea and combinations thereof.

8. A perfume particle according to any preceding claim, wherein the perfume ingredients comprise free perfume.

9. A perfume particle according to any preceding claim, wherein the perfume ingredients comprise encapsulated perfume.

10. A perfume particle according to any preceding claim, wherein the perfume particles further comprise a colourant.

11. A method of preparing a perfume particle composition according to claim 1, wherein the method comprises the steps of:

i. Obtaining a carrier ingredient comprising at least one ethoxylate derived from biomass;
ii. Melting said carrier material;
iii. Adding the perfume ingredients;
iv. Shaping and cooling the melt.

12. Use of a perfume particle according to claims 1 to 10 to reduce the carbon footprint of the perfume particle.

**Patentansprüche**

1. Duftstoffpartikelzusammensetzung, umfassend:

a) mindestens 10 Gew.-% eines Trägermaterials, ausgewählt unter Alkoholethoxylaten, Polyethylenglycolen und Kombinationen davon, welches mindestens eine aus Biomasse gewonnene Ethoxylateinheit umfasst; und
b) Duftstoffbestandteile,

wobei mindestens 50 Gew.-% der Ethoxylateinheiten im Bestandteil a) aus pflanzlicher Biomasse gewonnen werden.

2. Duftstoffpartikel nach Anspruch 1, wobei weniger als 10 Gew.-% der Ethoxylateinheiten im Bestandteil a) direkt aus unberührten fossilen Brennstoffquellen erhalten werden.

3. Duftstoffpartikel nach einem vorhergehenden Anspruch, wobei der Bestandteil a) ein Alkoholethoxylat ist.

4. Duftstoffpartikel nach einem vorhergehenden Anspruch, wobei der Bestandteil a) ein Polyethylenglycol mit einem Molekulargewicht von 2000 bis 20000 ist.

5. Duftstoffpartikel nach einem vorhergehenden Anspruch, wobei die Biomasse Cellulosematerialien darstellt.

6. Duftstoffpartikel nach einem vorhergehenden Anspruch, wobei das Trägermaterial, welches mindestens ein aus Biomasse gewonnenes Ethoxylat umfasst, einen prozentualen Anteil an modernem Kohlenstoff (pMC) von 50% bis 100% aufweist, der entsprechend den Angaben in der Beschreibung bestimmt wird.

7. Duftstoffpartikel nach einem vorhergehenden Anspruch, wobei das Duftstoffpartikel ein zusätzliches Trägermaterial umfasst, ausgewählt unter synthetischen Polymeren, die nicht aus Biomasse gewonnen werden, Proteinen, Sacchariden, Polysacchariden, wasserlöslichen oder wasserdispergierbaren Füllstoffen, Pflanzenseife, ethoxylierten nichtionischen Tensiden, die nicht aus Biomasse gewonnen werden, Harnstoff und Kombinationen.

8. Duftstoffpartikel nach einem vorhergehenden Anspruch, wobei die Duftstoffbestandteile freien Duftstoff umfassen.

**9.** Duftstoffpartikel nach einem vorhergehenden Anspruch, wobei die Duftstoffbestandteile eingekapselten Duftstoff umfassen.

**10.** Duftstoffpartikel nach einem vorhergehenden Anspruch, wobei die Duftstoffpartikel ferner ein Färbemittel umfassen.

**11.** Verfahren zur Herstellung einer Duftstoffpartikelzusammensetzung nach Anspruch 1, wobei das Verfahren die folgenden Schritte umfasst:

i. Erhalten eines Trägerbestandteils, welcher mindestens ein aus Biomasse gewonnenes Ethoxylat umfasst;
ii. Schmelzen des Trägermaterials;
iii. Hinzufügen der Duftstoffbestandteile;
iv. Formen und Abkühlen der Schmelze.

**12.** Verwendung eines Duftstoffpartikels nach den Ansprüchen 1 bis 10 zum Vermindern des $CO_2$-Fußabdrucks des Duftstoffpartikels.

**Revendications**

**1.** Composition de particule de parfum comprenant :

a) au moins 10 % en poids d'un matériau de support choisi parmi les alcools éthoxylés, les polyéthylèneglycols et leurs combinaisons, qui comprend au moins un motif d'éthoxylation dérivé de biomasse ; et
b) des ingrédients de parfum

dans laquelle au moins 50 % en poids des motifs d'éthoxylation dans l'ingrédient a) dérivent de biomasse végétale.

**2.** Particule de parfum selon la revendication 1, dans laquelle moins de 10 % en poids des motifs d'éthoxylation dans l'ingrédient a) sont obtenus directement à partir de sources de carburant fossile vierge.

**3.** Particule de parfum selon l'une quelconque des revendications précédentes, dans laquelle l'ingrédient a) est un alcool éthoxylé.

**4.** Particule de parfum selon l'une quelconque des revendications précédentes, dans laquelle l'ingrédient a) est un polyéthylèneglycol ayant une masse moléculaire de 2 000 à 20 000.

**5.** Particule de parfum selon l'une quelconque des revendications précédentes, dans laquelle la biomasse consiste en matériaux cellulosiques.

**6.** Particule de parfum selon l'une quelconque des revendications précédentes, dans laquelle le matériau de support comprenant au moins un produit d'éthoxylation dérivé de biomasse a un pourcentage de carbone moderne (pMC) de 50 % à 100 %, qui est déterminé conformément au fascicule dans la description.

**7.** Particule de parfum selon l'une quelconque des revendications précédentes, laquelle particule de parfum comprend un matériau de support additionnel, choisi parmi : les polymères synthétiques non dérivés de biomasse, les protéines, les saccharides, les polysaccharides, les charges solubles dans l'eau ou dispersibles dans l'eau, un savon végétal, les tensioactifs non-ioniques éthoxylés non dérivés de biomasse, l'urée, et leurs combinaisons.

**8.** Particule de parfum selon l'une quelconque des revendications précédentes, dans laquelle les ingrédients de parfum comprennent un parfum libre.

**9.** Particule de parfum selon l'une quelconque des revendications précédentes, dans laquelle les ingrédients de parfum comprennent un parfum encapsulé.

**10.** Particule de parfum selon l'une quelconque des revendications précédentes, lesquelles particules de parfum comprennent en outre un colorant.

**11.** Procédé de préparation d'une composition de particule de parfum selon la revendication 1, lequel procédé comprend

les étapes de :

i. l’obtention d’un ingrédient de support comprenant au moins un produit d’éthoxylation dérivé de biomasse ;
ii. la fonte dudit matériau de support ;
iii. l’addition des ingrédients de parfum ;
iv. la mise en forme et le refroidissement de la masse fondue.

**12.** Utilisation d’une particule de parfum selon les revendications 1 à 10 pour réduire l’empreinte carbone de la particule de parfum.

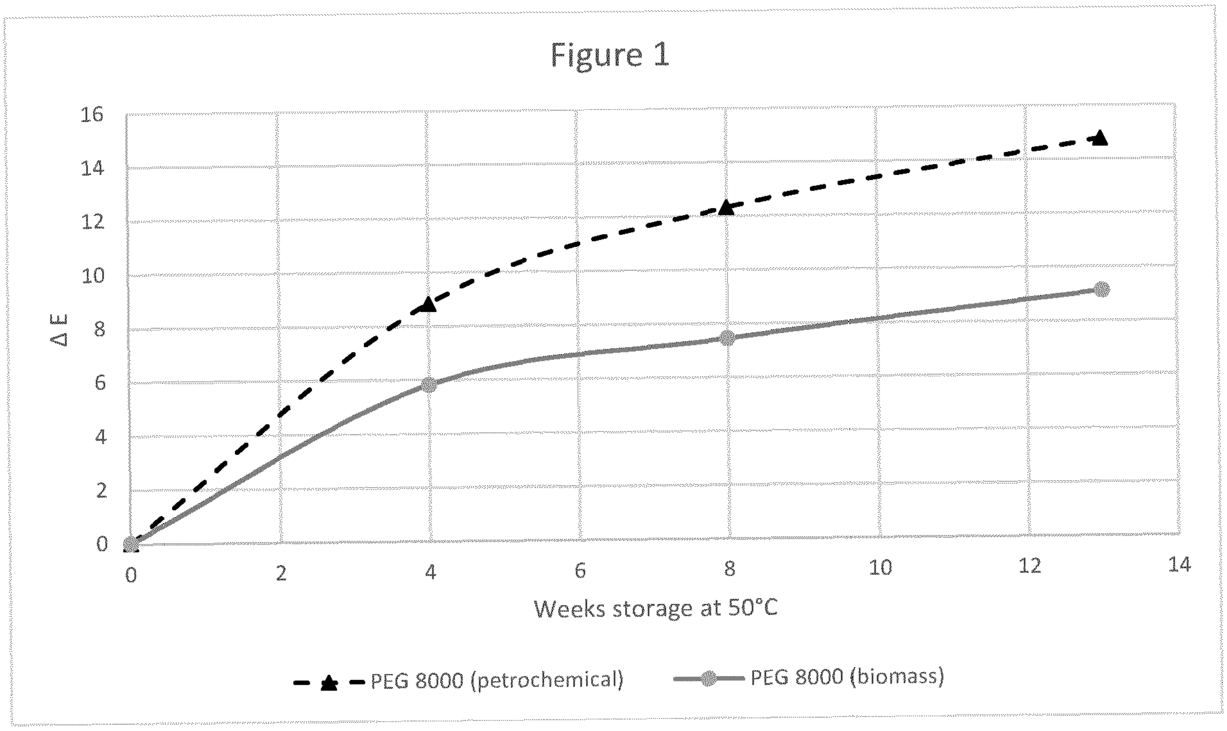
Figure 1
16
14
12
10
8
6
4
2
0
Δ E
0
2
4
6
8
10
12
14
Weeks storage at 50°C
PEG 8000 (petrochemical)
PEG 8000 (biomass)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019025216 A1


### Non-patent literature cited in the description


- Fenaroli's Handbook of Flavor Ingredients. CRC Press, 1975
- **M. B. JACOBS**. Synthetic Food Adjuncts. 1947
- **S. ARCTANDER**. *Perfume and Flavor Chemicals*, 1969
- Industrial Inorganic Pigments. Wiley-VCH, 2005
- Industrial Organic Pigments. Wiley-VCH, 2004
- Industrial Dyes. Wiley-VCH, 2003